# EUROPEAN PATENT APPLICATION

(11) **EP 3 714 817 A1**
(43) Date of publication of application: **30.09.2020**
(21) Application number: 19166257.6
(22) Date of filing: 29.03.2019
(51) Int. Cl.: A61B 17/3205, A61B 17/221

(54) **COLONOSCOPE FOR REMOVING COLORECTAL POLYPS**

(71) Applicant: Dokter Lode Van Overbeke bvba, 2800 Mechelen (BE)
(72) Inventor: Van Overbeke, Lode, 2800 Mechelen (BE)
(74) Representative: IP HILLS NV

(57) **Abstract**

According to an embodiment a polypectomy device is disclosed for removing a polyp (310) comprising an outer tube (220) comprising a distal end and a proximal end; and a slidably disposed wire (210) arranged in the outer tube and extending between the distal and proximal end comprising a snare loop (110) at the distal end; and controlling means operatively coupled to the wire at the proximal end; wherein the controlling means are configured to slide the wire such that the snare loop extends out the outer tube at the distal end in an open condition for placing the snare loop around the polyp; wherein the outer tube further comprises cutting means (222) at the distal end configured to scrape the polyp, and wherein the controlling means are further configured to, when the snare loop is in the open condition, slide the cutting means with respect to the outer tube towards the polyp for removing it.

## Description

### Field of the Invention

The present invention generally relates to medical devices. More particularly, the present disclosure relates to polypectomy devices for removal of gastrointestinal polyps, such as colorectal polyps.

### Background of the Invention

A gastrointestinal polyp such as a colorectal polyp, or in short, a polyp, is a fleshy growth occurring on the lining of the colon of a patient. It is an abnormal growth of tissue projecting from a mucous membrane, or in short mucosa. A polyp is either pedunculated, which means that it is attached to a gastrointestinal wall by a stalk, or sessile, which means that it grows directly from the gastrointestinal wall.

Since untreated polyps can develop into colorectal cancer, they are removed endoscopically through a colonoscopy. A colonoscope comprising a polypectomy device is used to remove polyps. The polypectomy device comprises at one end, the distal end, a snare loop. For removing pedunculated polyps, the snare loop is opened and passed over the polyp around the stalk thereof. Next, the loop of the snare is tightened to grip the polyp's stalk, and the polyp is pulled away from the gastrointestinal wall of the colon. Additionally, an electrical current may be passed through the snare loop to cut the polyp's stalk, providing electrocautery at the same time. This technique is also identified as a resection through hot snare polypectomy. With this technique large fragments of tissues may be cut present at big polyps.

When a stalk is absent, such as for sessile polyps, a sclerotherapy needle is used for injecting saline in the submucosa to elevate the mucosa thereby elevating the polyp. The elevated polyp may then be removed by the snare loop in a similar manner like the pedunculated polyps. Further, the elevating of the polyp is needed for reducing a risk of damaging the muscularis when using the hot snare polypectomy technique.

In US2006235433A1 such a polypectomy device is disclosed comprising an extendable and retractable snare loop. In US2018028219A1 another polypectomy device is disclosed which further comprises a retention member for holding a removed polyp for further analysing the polyp.

A drawback of removing a polyp by a snare loop is that besides mucosa also submucosa may need to be removed. Especially in the case of applying hot snare polypectomy the use of coagulation causes a damage of extra tissue resulting in deeper cuts. This further causes a risk of damaging muscularis close to the removed submucosa. Moreover, large submucosal blood-vessels may likewise be damaged resulting in a perforation and/or critical haemorrhage. Another drawback is that it is time consuming to elevate sessile polyps for removing them by a snare loop.

It is therefore an object of the present invention to alleviate the above drawbacks and to provide an improved device for removing polyps in an efficient manner and reducing the risk of damaging the muscularis and the submucosa.

### Summary of the Invention

This object is achieved, in a first aspect, by a polypectomy device for an endoscope configured to remove a polyp, the polypectomy device comprising:
- an outer tube comprising:
   ∘ a distal end and a proximal end; and
   ∘ a slidably disposed wire arranged in the outer tube and extending between the distal and proximal end comprising a snare loop at the distal end; and
- controlling means operatively coupled to the wire at the proximal end;
wherein the controlling means are configured to slide the wire such that the snare loop extends out the outer tube at the distal end in an open condition for placing the snare loop around the polyp;
CHARACTERIZED IN THAT
the outer tube further comprises cutting means at the distal end configured to scrape the polyp, and wherein the controlling means are further configured to, when the snare loop is in the open condition, slide the cutting means with respect to the outer tube towards the polyp for removing it.

The polypectomy device comprises an outer tube with a distal and a proximal end, a wire, and controlling means. The controlling means may, for example, be incorporated in a body, or in an assembly. The distal end is situated away from the controlling means, while the proximal end is situated close to the controlling means. The distal end is the part which is passed through an opening of a patient, such as the anus. This is, for example, performed by a colonoscope wherein the polypectomy device is inserted or plugged in, or when possible directly by the polypectomy device. The proximal end remains close to a medical doctor when performing a medical surgery. The medical doctor may thus be a surgeon, an internist, a gastroenterologist, or another person trained for performing such surgical operation. Next, the proximal end is situated outside the patient. Consequently, the outer tube extends between the proximal end and the distal end, and the controlling means are situated at the proximal end. The outer tube comprises a wire slidably disposable therein and likewise extending between the distal end and proximal end. Further, the wire comprises a snare loop at the distal end. Furthermore, the controlling means are operatively coupled to the wire at the proximal end and configured to control the wire. The snare loop at the distal end of the wire is configured for placing it around a polyp. This is performed by the controlling means which slides the wire in the direction from the proximal end to the distal end such that the snare loop extends outside the outer tube at the distal end. The snare loop extending out the outer tube is further identified as an open condition of the snare loop. In this open condition, the snare loop can be placed around a polyp when, for example, performing a surgery for removing a polyp.

The polypectomy device is characterized in that the outer tube further comprises cutting means at the distal end thereof. The cutting means are configured to scrape a polyp, like gastro-intestinal polyps, colorectal polyps, or other polyps present in, for example, the colon, stomach, or duodenum of the patient. To do so, the controlling means of the polypectomy device are configured to slide the cutting means out of the outer tube at the distal end.

When the snare loop is in the open condition, thus extending out the outer tube at the distal end, the cutting means may slide out of the outer tube. Thus, when the snare loop is placed around the polyp, the cutting means are slid by the controlling means towards the polyp. The polyp is then removed by the cooperation of the snare loop and the cutting means. The polyp is hold or retained by the snare loop and the cutting means scrape the polyp thereby removing it.

Since polyps may stay stuck when removing them with solely a cold snare loop, it is an advantage that the polyp can be removed by the aid of cutting means as an additional or complementary tool. There is thus no need of using electrocautery or electrocoagulation. This way, the polyp may be removed more efficiently. Furthermore, a risk of damaging the submucosal vessels or erroneously removing muscularis is reduced. Using the cutting means in combination with the snare loop, a polyp may be removed more precisely, thereby avoiding such a damage and/or removal.

It is further an advantage that by the cutting means, sessile polyps may be removed as well without having the need of injecting saline for an elevation. For removing unelevated sessile polyps, the snare loop is placed around such a polyp for retaining it and subsequently the cutting means scrape the polyp. Therefore, sessile polyps may be removed in a fast safe and efficient manner.

According to an embodiment, the cutting means are further configured to scrape or slice mucosa.

In other words, the cutting means are configured such that the polyp can be removed completely by slicing or scraping the mucosa. To do this, the snare loop is placed around the polyp, and the cutting means, or a part therefrom, is configured to slide underneath the snare loop. This way, the polyp and the mucosa underneath the polyp is scraped or sliced. The submucosa underneath the mucosa will remain untouched, thereby avoiding a risk of haemorrhage or bleeding. Further, likewise a risk of damaging muscularis at the spot of the polyp is reduced as well.

According to an embodiment, the cutting means comprises a hardened ending at the distal end of the outer tube.

The distal end of the outer tube is hardened such that it becomes applicable for scraping and/or slicing the polyp. In other words, the cutting means comprises the distal end of the outer tube which is slidable towards the polyp when the snare loop is placed around it. In case, the hardened distal end may also be configured for scraping or slicing mucosa.

Thus, when the snare loop is placed around the polyp, this is in the open condition, the controlling means are configured to slide the outer tube towards the snare loop, and therefore towards the polyp. The polyp is then retained by the snare loop and the outer tube with the hardened ending removes the polyp.

Differently formulated, the controlling means are configured to, firstly, slide the snare loop outside the outer tube at the distal end for placing it around the polyp. Secondly, the outer tube is slid towards the snare loop. In this second condition, the outer tube houses the snare loop when the cutting means have removed the polyp, which is a similar situation compared to an initial one. However, a difference between the initial situation, this is housing the snare loop when inserting the polypectomy device into the patient, is that the outer tube is further or deeper inserted. Hence, at the proximal end a part of the outer tube remains available such that the controlling means can perform this movement, this is further inserting the outer tube into the patient.

The hardened ending may, for example, comprise the material wherefrom the entire outer tube is fabricated. At the distal end, the material is then hardened, while at the other part, this is towards the proximal end, the material of the outer tube remains flexible.

Alternatively, a part of the outer tube at the distal end may comprises a different material compared to the other part, this is towards the proximal end. At the boundary between the different materials, the materials are joint such that the outer tube comprises a unity. This way, the manoeuvrability or workability is assured.

According to an embodiment, the cutting means comprises a sharp edge at the distal end of the outer tube.

The outer tube may, for example, comprise a flexible material whereby at the distal end, the material is sharpened. This may be performed by a bevelled edge whereby the tip of the distal end is configured to scrape or slice a polyp, and in case mucosa. The sharpening of the distal end may also be performed by the hardened material. The hardened material at the distal end then comprises a bevelled edge, or a protrusion comprising a thickness suitable for scraping the polyp. This way, the sharp edge forms a knife suitable for removing the polyp.

According to an embodiment, the cutting means comprises a ring member configured to be placed over the outer tube at the distal end.

The ring member is configured such that it can scrape the polyp. The ring member may comprise the same material as the outer tube, or alternatively another material, such as the hardened one. In both cases, the cutting means and the outer tube are two elements. By placing the ring member over the outer tube, the two elements are joined together. This joined configuration is then configured to slice the polyp, this is the outer tube slides the ring member, while the ring member performs the scraping.

The ring member and the outer tube may be configured in such a way that, when the ring member is placed over the outer tube, the diameter of the outer tube and the ring member remains the same over the axial length. In other words, the outer tube then comprises a smaller diameter at the distal end compared to the other part thereof. The ring member is placed over this smaller diameter, whereby the ring member itself has an outer diameter corresponding to the diameter of the outer tube towards the proximal end. The variation of the diameter of the outer tube at the distal end may be tapered or angulated. The ring member may then comprise a corresponding tapered or angulated inner diameter in an opposite direction such that they are steadily joinable.

According to a preferred embodiment, the cutting means comprises a slidably disposed inner tube arranged in the outer tube, and the controlling means are configured to slide the inner tube out of the outer tube.

In this embodiment, the polypectomy device comprises the outer tube, which further comprises an inner tube. The inner tube comprises the cutting means. In other words, the inner tube at the distal end is configured such that it can slice the polyp, or in case mucosa.

The inner tube is further configured such that it is slidable with respect to the outer tube. The inner tube can then slide outside the outer tube by use of the controlling means. Thus, when performing a surgery, firstly the snare loop is placed around the polyp. Next, the controlling means slide the cutting means comprising the inner tube out of the outer tube. In other words, the outer tube remains at a steady distance from the polyp, and the inner tube slides towards the polyps. Thirdly, the polyp may then be removed by use of the snare loop and the cutting means.

The inner tube comprises a hollow tube or comprises a solid body. Alternatively, the inner tube comprises at the distal end a solid body, and between the solid body and the controlling means a wire for controlling the solid body. The solid body is then configured as cutting means by, for example, a sharpened edge.

According to an embodiment the inner tube is configured to house the snare loop.

Preferably, the inner tube comprising the cutting means houses the snare loop. The polypectomy thus comprises an outer tube housing an inner tube, and the inner tube houses the snare loop. This way, the different components are positioned concentrically. This improves the controllability of the polypectomy device since the mechanical characteristics in the radial direction over the axis are balanced. Differently formulated, by positioning the elements concentrically, the presence of an unbalance is avoided thereby improving the overall controllability of the device.

According to an embodiment, the controlling means comprises securing means configured to block the cutting means when the snare loop is not in the open condition.

When the snare loop is not in the open condition, thus still housed by the outer tube, or in case the inner tube, the cutting means are blocked. This way it is avoided that a surgeon erroneously uses the cutting means prior to the use of the snare loop.

When a surgeon initiates a surgery, the polypectomy device is inserted into an opening of the patient, for example through the anus. The distal end is then further inserted through the colon. During this preparation step prior to any removal of a polyp, it must be avoided that the colon is damaged. Thus, the blocking means prevents the cutting means extending out of the outer tube when the snare loop is still housed.

According to an embodiment, the polypectomy device further comprises a protective tube for housing the outer tube.

Additionally, the outer tube may further be protected by a protective tube. The protective tube comprises, for example, a protective layer which may be removed prior to the use of the polypectomy device. This way, the polypectomy device may be transported and stocked in a safe and clean manner prior to its use.

According to an embodiment, the protective tube is configured to slide the outer tube.

Alternatively, the protective layer may also be composed or designed in such a way that it remains wrapped around the outer tube when the polypectomy device is in use. This way, there is no need to remove the protective layer such that the device can be prepared in a fast manner for using it. Furthermore, the protective tube provides a protective layer for the working channel of an endoscope when used therein. The working channel will then not be damaged by the cutting means.

The wire comprising the snare loop at the distal end comprises, according to an embodiment, nylon.

A polyp may be removed by the polypectomy device through the cooperation of the wire comprising the snare loop and the cutting means. Because of this, the wire and accompanying snare loop may comprise nylon instead of, for example, a metal. This way, the polypectomy device may be fabricated in an efficient manner, since there is no need on processing and treating a metal suitable to be used in a polypectomy device.

Another advantage of removing a polyp by the combination of the snare loop and the cutting means is that the removal may be performed by cold snare polypectomy. Therefore, there is no need of passing an electrical current through the wire, and thus no need of using a metal as the wire. Instead, nylon may be used for the wire and the snare loop.

According to an embodiment, the cutting means comprise a metal.

The cutting means at the distal end of the outer tube may comprise a metal treated and completed to perform cutting or scraping acts. By using a metal for the cutting means, the cutting characteristics may be adapted to the needs in an efficient manner, since a metal is easily adaptable through, for example, sharpening or polishing operations.

According to another embodiment, the cutting means comprise a fibre.

Alternatively, the cutting means comprises a fibre, such as, for example, aramid fibres, carbon fibres, and/or other fibres suitable for the cutting or scraping acts.

According to a second aspect, the invention relates to an endoscope comprising the polypectomy device according to the first aspect.

The endoscope is configured such that is may comprise the polypectomy device according to the first aspect. An instrumentation channel of the endoscope is thus adapted to house the polypectomy device. The endoscope is further configured such that the wire comprising the snare loop and the cutting means may be handled and controlled in an efficient and effective manner.

The endoscope may further be configured such that, in case, the blocking means blocks the cutting means when the snare loop is still housed by the outer tube.

The endoscope comprises one of the group of a colonoscope, a gastroscope, and/or a duodenoscope.

In other words, the endoscope is suitable for a variety of medical procedures.

### Brief Description of the Drawings

Some example embodiments will now be described with reference to the accompanying drawings.
Fig. 1A illustrates a polypectomy device according to an embodiment of the invention; and
Fig. 1B illustrates two detailed views of the distal end of the polypectomy device of Fig. 1A according to two example embodiments; and
Fig. 2 illustrates three positions of the cutting means and the snare loop at the distal end of the polypectomy device of Fig. 1A; and
Fig. 3 illustrates a first step for removing a pedunculated polyp by the polypectomy device according to an embodiment of the invention; and
Fig. 4 illustrates a second step following the first step of Fig. 3; and
Fig. 5 illustrates a third step following the second step of Fig. 4; and
Fig. 6 illustrates a fourth step following the third step of Fig. 5 whereby the polyp is removed; and
Fig. 7 illustrates a step for removing a sessile polyp according to an embodiment of the invention; and
Fig. 8 illustrates a human intestine with a colonoscope therein.

### Detailed Description of Embodiment(s)

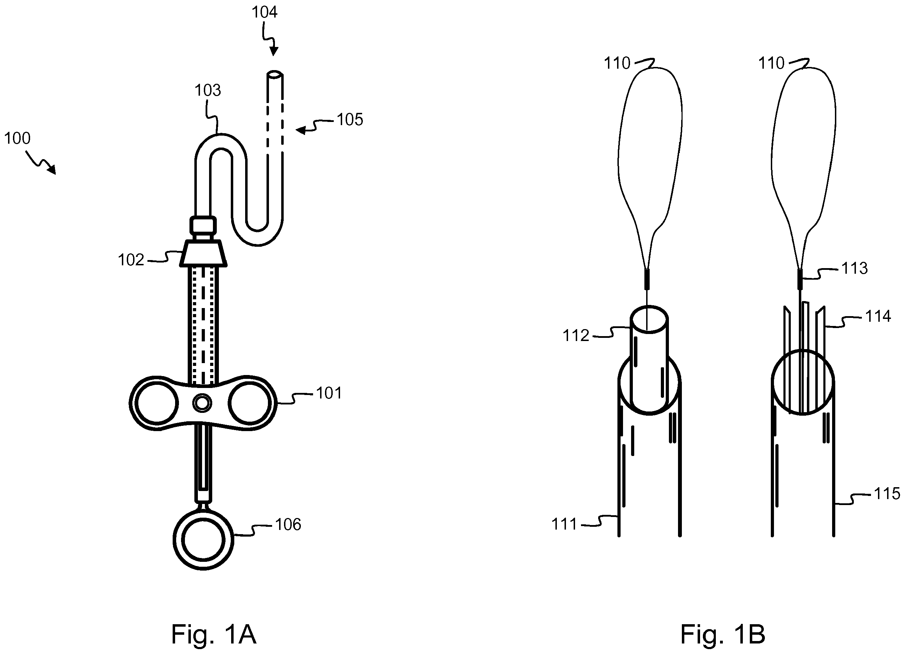

Fig. 1A illustrates a polypectomy device according to an example embodiment of the invention. The polypectomy device 100 comprises controlling means 101, 106, and an outer tube 103 extending from the proximal end 102 to the distal end 104. The outer tube 103 comprises a flexible material and is suitable for inserting it in an opening of a patient, like for example the anus or the throat. The insertion is performed by the distal end 104 and the proximal end 102 remains fixed on the controlling means and close to the person performing the surgery. The length of the tube 103 is adapted to the type of surgery for which the polypectomy device 100 will be used for. This is illustrated by the dotted lines 105 indicating that the length of the tube 103 is longer then could be interpreted from the illustration of Fig. 1A alone.

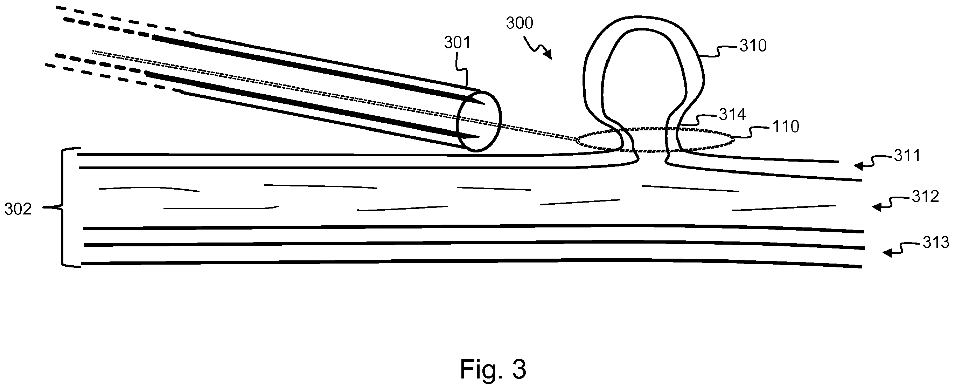

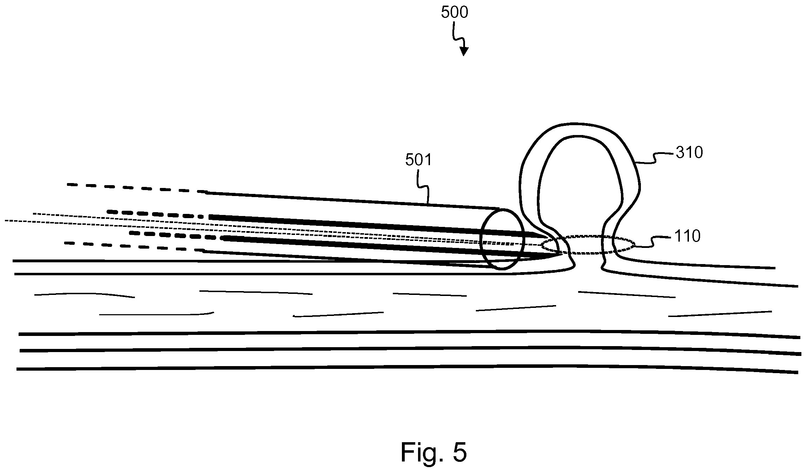

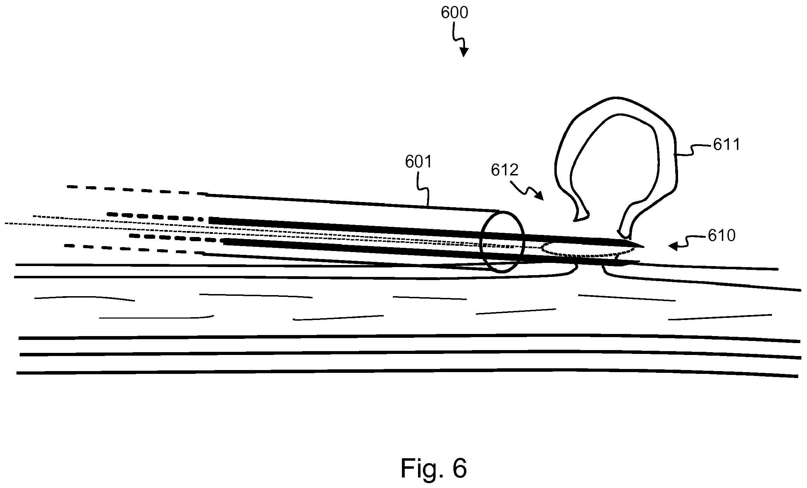

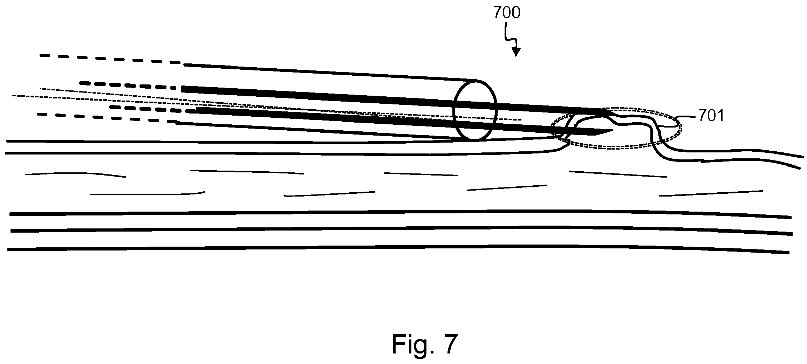

The polypectomy device 100 is configured to remove a polyp, like for example gastrointestinal polyps like a colorectal polyp, or polyps in the stomach or at the duodenum of a patient. A polyp may further be classified as a pedunculated polyp or a sessile polyp. A pedunculated polyp is illustrated in Fig. 3 to Fig. 6 which illustrates steps performed for removing the pedunculated polyp 310 by the polypectomy device 100. In Fig. 7 a sessile polyp is illustrated. A difference between these two types of polyps is that a pedunculated polyp 310 comprises a stalk 314, whereas a sessile polyp 701 doesn't.

For removing a polyp, the polypectomy device 100 comprises a snare loop and cutting means. This is further illustrated in Fig. 1B. The snare loop 110 comprises a material suitable for gripping or clasping a polyp as illustrated in Fig. 3 to Fig. 6 and Fig. 7. The material is for example a metal, nylon, or another material having a strength suitable for gripping a polyp. To form the loop 110, a connection 113 is made. This connection 113 comprises for example a metal or another material suitable for holding the snare loop 110 at one end thereby forming it 110. The polypectomy device 100 further comprises cutting means. The cutting means may be constructed in different shapes, such as a tube 112 or as one or more cutting blades 114. The tube 112 may further be protected by a protective tube 111. Likewise, the blades 114 may be protected by a tube 115 as well. Alternatively, the distal end 104 may comprises a hardened material comprising the cutting means, for example the outer tube 111, 115 itself hardened at the end 104 therefrom which then forms the cutting means of the polypectomy device 100.

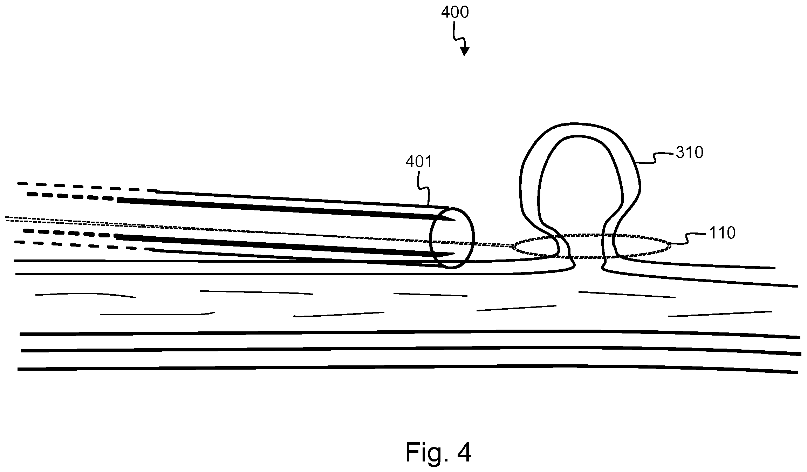

The cutting means 112, 114 are configured to scrape of slice a polyp 310, 701. This scraping or slicing will further be illustrated with reference to Fig. 3 to Fig. 6. In Fig. 3 a cross-section of a gastrointestinal wall 302 is further illustrated. The gastrointestinal wall 302 comprises mucosa 311, submucosa 312 and muscularis 313. These three layers 311, 312, 313 are likewise illustrated in Fig. 4, Fig. 5, Fig. 6 and Fig. 7.

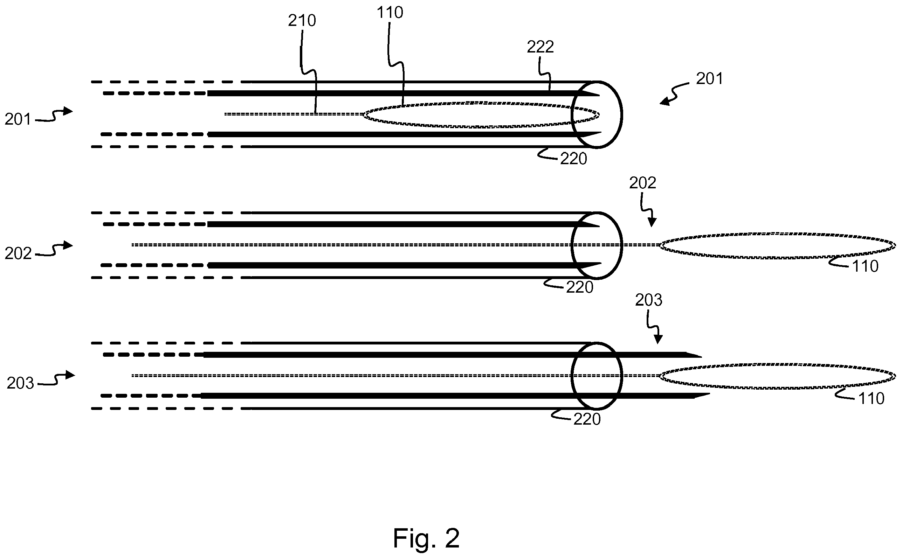

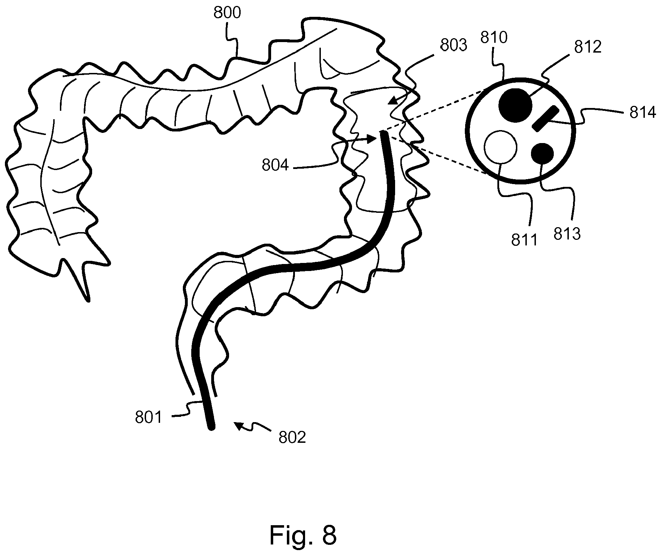

The gastrointestinal wall 302 is present in a human intestine 800 as illustrated in Fig. 8. Next, a polyp 310, 701 may be present in the human intestine 800, for example in the zone 803. To remove a polyp in the human intestine 800 in the zone 803 a colonoscope 801 is inserted through opening 802 which illustrates the anus of a patient. A cross-section 810 of the colonoscope 801 is further illustrated comprising a working channel or instrument port 811, a lens 812, a light source 813 and an irrigation 814. In the colonoscope 801, in particular in the working channel 811, the polypectomy device 100 with the distal end 104 thereof is inserted. When the ending 804 of the colonoscope 801 is located in zone 803 a medical doctor such as a surgeon, an internist, a gastroenterologist or another person trained for performing surgical operations will remove the polyp 310, 701. Several consecutive steps are performed by the medical doctor when removing a polyp and these steps will further be illustrated with references to Fig. 2 and to Fig. 3 to Fig. 6.

Fig. 2 illustrates three distinct conditions of the disclosed polypectomy device 100 at the distal end 104. In a first condition 201, the snare loop 110 and the cutting means referred by reference 222 are housed by the tube referred by reference 220. The cutting means 222 may thus correspond to the cutting tube 112 or to the cutting blades 114. The tube 220 may correspond to the tube 111 or to the tube 115.

The controlling means 101, 106 of the disclosed polypectomy device 110 is configured to slide the snare loop 110 out of the tube 220. This is illustrated by condition 202 wherein the snare loop 110 extends out the tube 220. The sliding may be performed by a wire 210 extending from the distal end 104 to the proximal end 102 in the tube 103, 220. The wire 210 thus pushes the connection 113 out of the tube 103, 220, thereby pushing out the snare loop 110. Finally, the controlling means 101, 106 is further configured to slide the cutting means 222 out of the tube 103, 220. This is illustrated by condition 203. This may likewise be performed by another wire between the cutting means 222 at the distal end 104 and the controlling means 101, 106 at the proximal end 102.

In a preferred embodiment, the controlling means 101, 106 is configured such that the cutting means 222 can only slide out of the tube 103, 220 when the snare loop 110 is already slid out. This improves the safety of use. For example, when the colonoscope 801 is inserted in the opening 802, the distal end 104 first has to reach the zone 803, and the snare loop 110 first has to slid out before the cutting means 222 can extend out of the tube 103, 220.

The condition 201 corresponds to the initiation step when the distal end 104 is inserted in the patient. This may be performed directly by the tube 103, or by the colonoscope 801 wherein the tube 103 is inserted. In the latter situation, the ending 804 of the colonoscope 801 is inserted in the human intestine 800 until the ending 804 is situated in zone 803.

In the gastrointestinal wall 302 a polyp may occur on the lining of the colon. As already highlighted, the polyp may either be a pedunculated polyp 310 or a sessile polyp 701. To remove a polyp, for example present in zone 803, the several consecutive steps as already highlighted in Fig. 2 are performed. These steps will further be illustrated with references to Fig. 3 to Fig. 6. These latter steps correspond to the occurrence that the ending 804 of the colonoscope 801 in situated in zone 804. Hereto, the person performing the surgery uses the lens 812 and light source 813 such that he or she can observe the different manipulations.

The steps illustrated in Fig. 3 to Fig. 6 comprise the polypectomy device 100 without the colonoscope 801 but it should be further understood that the steps may likewise correspond to a situation wherein the tube 103 of the polypectomy device 100 is inserted in the working channel 811 of the colonoscope 801.

With reference to Fig. 3, a first step is approaching the polyp 310 by the distal end 104 of the polypectomy device 100. The snare loop 110 is slid out of the polypectomy device 100 as illustrated in condition 202 and wrapped 300 around the stalk 314 of the polyp 310. The cutting means 222 remain in the tube 103, 220, which is illustrated by reference 301. In a next step 400, to remove the polyp 310, the direction of the tube 103 or colonoscope 801 is adapted such that the cutting means 222 points to the stalk 314. This is illustrated by reference 401. Subsequently 500, the polyp 310 will be removed by the cutting means 220 which are slid out of the tube 103 by the controlling means 101, 106. This is illustrated by reference 501. The cutting means 222 then scrape of slice the polyp 310 by its stalk 314 until polyp 310 is removed. This is illustrated by step 600. The polyp 611 is removed 612 by slicing 610 the stalk 314. The last step 601 thus corresponds to step 203. Next, the cutting means 222 and snare loop 110 may again be housed by the tube 220, thus again back to the initial step 201. The surgeon then continues identifying another polyp and repeats the steps 300, 400, 500 and 600. The other polyp may, for example, be a sessile polyp 701. Likewise, the snare loop 110 is slid out of the tube 220 such that it 110 envelops the sessile polyp 701 as illustrated by reference 700. The cutting means 222 are then slid out and the sessile polyp 701 is scraped of sliced to remove it.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above

## Claims

1. A polypectomy device (100) for an endoscope (801) configured to remove a polyp (310, 701), the polypectomy device (100) comprising:
- an outer tube (103, 220) comprising:
∘ a distal end (104) and a proximal end (104); and
∘ a slidably disposed wire (210) arranged in the outer tube (103, 220) and extending between the distal (104) and proximal end (104) comprising a snare loop (110) at the distal end (104); and
- controlling means (101, 106) operatively coupled to the wire (210) at the proximal end (102);
wherein the controlling means (101, 106) are configured to slide the wire (210) such that the snare loop (110) extends (202) out the outer tube (103, 220) at the distal end (104) in an open condition (202) for placing (300) the snare loop (110) around the polyp (310, 701);
**CHARACTERIZED IN THAT**
the outer tube (103) further comprises cutting means (112, 114, 222) at the distal end (104) configured to scrape the polyp (310, 701), and wherein the controlling means (101, 106) are further configured to, when the snare loop (110) is in the open condition (202), slide (203) the cutting means (112, 114, 222) with respect to the outer tube (103, 220) towards the polyp (310, 701) for removing (600) it.

2. The polypectomy device (100) according to claim 1, wherein the cutting means (112, 114, 222) are further configured to scrape or slice mucosa (311).

3. The polypectomy device (100) according to one of the preceding claims, wherein the cutting means (112, 114, 222) comprises a hardened ending at the distal end (104) of the outer tube (103).

4. The polypectomy device (100) according to one of the preceding claims, wherein the cutting means (112, 114, 222) comprises a sharp edge at the distal end (104) of the outer tube (103).

5. The polypectomy device (100) according to one of the preceding claims, wherein the cutting means (112, 114, 222) comprises a ring member configured to be placed over the outer tube (103) at the distal end (104).

6. The polypectomy device (100) according to claim 1, wherein the cutting means (112, 114, 222) comprise a slidably disposed inner tube arranged in the outer tube (103); and wherein the controlling means (101, 106) are further configured to slide the inner tube out of the outer tube (103).

7. The polypectomy device (100) according to claim 6, wherein the inner tube is configured to house the snare loop (110).

8. The polypectomy device (100) according to one of the preceding claims, wherein the controlling means (101, 106) comprises securing means configured to block the cutting means (112, 114, 222) when the snare loop (110) is not in the open condition (202).

9. The polypectomy device (100) according to one of the preceding claims further comprising a protective tube for housing the outer tube (103).

10. The polypectomy device (100) according to claim 9, wherein the protective tube is configured to slide the outer tube (103).

11. The polypectomy device (100) according to one of the preceding claims, wherein wire (210) comprises nylon.

12. The polypectomy device (100) according to one of the preceding claims, wherein the cutting means (112, 114, 222) comprise a metal.

13. The polypectomy device (100) according to one of the preceding claims, wherein the cutting means (112, 114, 222) comprise a fibre.

14. An endoscope (801) comprising the polypectomy device (100) according to one of the preceding claims.

15. The endoscope (801) according to claim 12 comprising one of the group of a colonoscope, a gastroscope, and/or a duodenoscope
